# EUROPÄISCHE PATENTSCHRIFT

(11) **EP 2 446 810 B1**
(45) Veröffentlichungstag und Bekanntmachung des Hinweises auf die Patenterteilung: **16.03.2016**
(21) Anmeldenummer: 11186373.4
(22) Anmeldetag: 24.10.2011
(51) Int. Cl.: A61B 1/07, G02B 23/24, A61B 1/00

(54) **Endoskop mit einstellbarer Blickrichtung**
Endoscope with adjustable view angle
Endoscope à direction d'observation réglable

(30) Priorität: 02.11.2010 DE 102010050011
(43) Veröffentlichungstag der Anmeldung: 02.05.2012
(73) Patentinhaber: Karl Storz GmbH & Co. KG, 78532 Tuttlingen (DE)
(72) Erfinder: Dahmen, Jan, 78532 Tuttlingen (DE); Dr. Lei, Fang, 78532 Tuttlingen (DE)

(56) Entgegenhaltungen:
- EP-A1- 2 228 002
- EP-A2- 2 062 545
- DE-A1- 2 428 913
- DE-A1- 10 222 686
- US-A- 5 460 168
- US-A- 6 053 862
- US-A1- 2002 107 448
- US-A1- 2002 188 204

## Beschreibung

Die vorliegende Erfindung bezieht sich auf ein Endoskop mit einstellbarer Blickrichtung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das gesamte Sichtfeld bzw. Blickfeld ausreichend ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf. Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die außerhalb des Sichtfelds liegen. Eine unerwünschte Annäherung des distalen Endes des Endoskops an diese Gegenstände und eine resultierende Bestrahlung dieser Gegenstände mit einer zu hohen Strahlungsleistung fallen deshalb nicht auf.

Ein weiterer Nachteil besteht darin, dass auch Beleuchtungslicht, das außerhalb des Sichtfelds abgestrahlt wird, von Gegenständen oder opaken Medien gestreut oder reflektiert werden kann. Das reflektierte oder gestreute Beleuchtungslicht kann direkt oder indirekt in den Beobachtungsstrahlengang gelangen. Dadurch können Kontraste und vor allem in dunklen Bildbereichen die Unterscheidbarkeit von Gegenständen verringert werden.

Ein weiterer Nachteil rührt daher, dass die Beleuchtungsstärke bzw. die Intensität des Beleuchtungslichts in der Richtung, in der die Blickrichtung variiert werden kann (oft auch als vertikale Richtung bezeichnet) im Wesentlichen konstant ist, während sie in der dazu senkrechten Richtung (oft auch als horizontale Richtung bezeichnet) in der Regel zum Rand des Sichtfelds hin leicht abnimmt. Von Endoskopen mit feststehender Blickrichtung sind Benutzer jedoch in der Regel eine Beleuchtungsstärke gewohnt, die sowohl in horizontaler als auch in vertikaler Richtung zum Rand des Sichtfelds hin leicht abnimmt. Die in vertikaler Richtung konstante Beleuchtungsstärke kann deshalb als irritierend empfunden werden.

In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Die Erfinder der vorliegenden Erfindung haben jedoch festgestellt, dass die Verteilung des Beleuchtungslichts innerhalb des Sichtfelds mit der beschriebenen Anordnung von Prismen in vielen Fällen unbefriedigend ist.

In der DE2428913 A1 ist ein Endoskop beschrieben, in dessen distalem Ende längsweise flexible Lichtleitmittel vorgesehen sind, die von einer flexiblen Röhre umgeben sind, sich mit ihrem vorderen Ende bis vor die Fensteröffnung erstrecken und in einer licht-durchlassenden Ebene enden, die durch die Fensteröffnung auf ein Gesichtsfeld in der Körperhöhle gerichtet ist. Dabei ist das vordere Ende der Lichtleitmittel in einem bewegbaren Körper befestigt, und ist für ein Verschwenken des bewegbaren Körpers an seinem distalen Ende ein Betätigungsdraht vorgesehen, wobei ein Ende des Betätigungsdrahts am bewegbaren Körper so befestigt ist, dass bei Betätigung der Betätigungsmittel von der Kontrolleinheit aus die lichtdurchlassende Ebene des vorderen Endes der Lichtleitmittel auf ein anderes Gesichtsfeld in der, Körperhöhle gerichtet wird.

Der Gegenstand dieser Patentanmeldung gewährleistet allerdings nicht, dass das Gesichtsfeld und der beleuchtete Bereich ausreichend genau auf den zu beobachtenden Operationsbereich eingestellt werden können.

Eine Aufgabe der vorliegenden Erfindung besteht darin, ein verbessertes Endoskop mit einstellbarer Blickrichtung zu schaffen.

Diese Aufgabe wird durch den Gegenstand des unabhängigen Anspruchs gelöst.

Weiterbildungen sind in den abhängigen Ansprüchen angegeben.

Ausführungsformen der vorliegenden Erfindung beruhen auf der Idee, zur Anpassung der Beleuchtungsrichtung an eine einstellbare Blickrichtung eines Endoskops das distale Ende eines Lichtleiters abhängig von der Beleuchtungsrichtung bzw. der Blickrichtung zu verformen. Damit ist es möglich, nur das Sichtfeld in der momentan eingestellten Blickrichtung zu beleuchten oder auszuleuchten. Dies reduziert die erforderliche Gesamtstrahlungsleistung, das Risiko von photothermischen oder photochemischen Schäden und die Intensität von Streulicht, das den Bildkontrast reduziert. Ferner kann bei entsprechender Ausgestaltung der Lichtaustrittseinrichtung unabhängig von der Beleuchtungsrichtung eine von Verwendern als angenehm empfundene Verteilung des Beleuchtungslichts erzielt werden, bei der die Beleuchtungsstärke insbesondere zu allen Rändern des Sichtfelds hin leicht abnimmt.

Ein Endoskop mit einstellbarer Blickrichtung umfasst eine Lichtaustrittseinrichtung am distalen Ende des Endoskops zum Abstrahlen von Beleuchtungslicht in eine Beleuchtungsrichtung, wobei die Lichtaustrittseinrichtung zum Einstellen der Beleuchtungsrichtung relativ zum Endoskop bewegbar ist, und einen Lichtleiter zum Übertragen von Beleuchtungslicht zur Lichtaustrittseinrichtung, wobei ein flexibler Abschnitt des Lichtleiters dazu ausgebildet und angeordnet ist, elastisch verformt zu werden, wenn die Lichtaustrittseinrichtung bewegt wird.

Die Blickrichtung des Endoskops ist insbesondere um eine Schwenkachse schwenkbar, die senkrecht zur Längsachse des Schafts des Endoskops ist. Die Längsachse des Endoskops ist insbesondere die Längsachse des Schafts. Im Fall eines starren geraden Schafts ist die Längsachse des Schafts die Gerade, auf der die Mittelpunkte der Querschnittsflächen des Schafts liegen. Im Fall eines flexiblen Schafts ist die Längsachse des Endoskops die Längsachse des distalen Endes des Schafts, also die Gerade, auf der Mittelpunkte der Querschnittsflächen des Schafts nahe dessen distalem Ende liegen.

Die Lichtaustrittseinrichtung ist insbesondere ausgebildet, um synchron mit der Blickrichtung des Endoskops bewegt, insbesondere geschwenkt, zu werden. Dazu ist beispielsweise eine Führungseinrichtung zum Führen der Lichtaustrittseinrichtung so mit einer Einrichtung zum Einstellen der Blickrichtung des Endoskops mechanisch gekoppelt, dass die Beleuchtungsrichtung jederzeit im Wesentlichen der Blickrichtung entspricht.

Der Lichtleiter umfasst insbesondere eine oder mehrere lichtleitende Fasern, beispielsweise Glasfasern oder Kunststofffasern, zum Übertragen von Beleuchtungslicht. Der flexible Abschnitt des Lichtleiters liegt insbesondere am distalen Ende des Endoskops. Beispielsweise schließt sich der flexible Abschnitt des Lichtleiters unmittelbar an die Lichtaustrittseinrichtung oder an eine Fassung zum Halten des distalen Endes des Lichtleiters an. Der gesamte Lichtleiter kann flexibel sein. Mit einer elastischen Verformung des flexiblen Abschnitts ist eine rein elastische oder im Wesentlichen elastische Verformung gemeint, wobei ein plastischer Anteil der Verformung nicht ausgeschlossen ist.

Die Lichtaustrittseinrichtung ist im einfachsten Fall eine als Lichtaustrittsfläche gestaltete distale Stirnfläche des Lichtleiters. Alternativ umfasst die Lichtaustrittseinrichtung eine oder mehrere Linsen, Spiegel, Prismen oder andere optische Elemente zur Formung eines am distalen Ende aus dem Lichtleiter ausgekoppelten Lichtbündels.

Insbesondere bei einer Ausbildung der Lichtaustrittseinrichtung als Lichtaustrittsfläche am distalen Ende des Lichtleiters ist ein hoher Wirkungsgrad erzielbar. Verluste durch Reflexionen an Lichteintrittsflächen und Lichtaustrittsflächen von Prismen, wie sie gemäß der DE 600 15 375 T2 verwendet werden, entfallen.

Ein weiterer Vorteil besteht in einem reduzierten Aufwand für die Fertigung, insbesondere die Justage, des Beleuchtungsstrahlengangs zur Übertragung von Beleuchtungslicht. Während beispielsweise bei der Verwendung von Prismen gemäß der DE 600 15 375 T2 jedes einzelne optische Element präzise gelagert und dabei sowohl hinsichtlich translatorischer als auch hinsichtlich rotatorischer Freiheitsgrade präzise ausgerichtet sein muss, sind vorliegend lediglich das proximale Ende und das distale Ende des Lichtleiters genau zu justieren. Dabei hat bei Ausbildung der Lichtaustrittseinrichtung als Lichtaustrittsfläche am distalen Ende des Lichtleiters selbst eine rein translatorische Fehljustage einen allenfalls geringen Einfluss auf die Qualität der Beleuchtung eines mittels des Endoskops betrachteten Gegenstands. Lediglich hinsichtlich rotatorischer Freiheitsgrade kann eine präzise Justage der distalen Lichtaustrittsfläche des Lichtleiters vorteilhaft oder erforderlich sein.

Ein weiterer Vorteil der Verwendung eines Lichtleiters, dessen flexibler Abschnitt bei Änderung der Blickrichtung elastisch verformt wird, kann in einem vergleichsweise geringen Raumbedarf bestehen. Insbesondere kann der Querschnitt des Lichtleiters kleiner sein als Prismen, die beispielsweise gemäß der DE 600 15 375 T2 Verwendung finden. Unter räumlich beengten Verhältnissen, wie sie typischerweise am distalen Ende in einem Endoskop vorliegen, schafft die Verwendung eines flexiblen Lichtleiters zusätzliche gestalterische Freiheitsgrade.

Bei einem Endoskop, wie es hier beschrieben ist, kann der Lichtleiter ein Bündel von lichtleitenden Fasern umfassen, wobei ein flexibler Mantel, in dem das Bündel von lichtleitenden Fasern im Bereich des flexiblen Abschnitts angeordnet ist, vorgesehen ist.

Der flexible Mantel ist beispielsweise ein Schrumpfschlauch oder ein anderer Schlauch mit kreisförmigem oder ovalem oder anderem Querschnitt. Der flexible Mantel weist beispielsweise Gummi, Silikon, ein Elastomer oder einen anderen elastischen Kunststoff auf. Das distale Ende des flexiblen Mantels schließt insbesondere unmittelbar an die Lichtaustrittseinrichtung oder an eine Fassung zum Halten des distalen Endes des Lichtleiters an oder ist mit der Lichtaustrittseinrichtung bzw. der Fassung dauerhaft mechanisch verbunden.

Der flexible Mantel kann den Lichtleiter in dessen flexiblem Abschnitt stützen, um beispielsweise eine gleichmäßige Verformung des Lichtleiters zu begünstigen und ein Knicken bzw. eine lokal stark erhöhte Krümmung des Lichtleiters zu vermeiden. Der flexible Mantel kann den Lichtleiter ferner vor Verschleiß durch Reiben an Kanten oder Flächen und vor anderen mechanischen Schäden schützen. Somit kann der flexible Mantel das Risiko eines Defekts am flexiblen Abschnitt des Lichtleiters deutlich mindern und die Lebensdauer des flexiblen Abschnitts des Lichtleiters und damit des gesamten Endoskops deutlich erhöhen.

Ein Endoskop mit einem flexiblen Mantel im Bereich des flexiblen Abschnitts des Lichtleiters, wie es hier beschrieben ist, kann ein Gleitmittel aufweisen, das in dem Mantel angeordnet ist, um zumindest entweder Reibung innerhalb des Bündels von lichtleitenden Fasern oder Reibung zwischen lichtleitenden Fasern und dem flexiblen Mantel zu mindern.

Das Gleitmittel ist insbesondere pulverförmig (beispielsweise Talkum), flüssig oder sehr duktil und wird durch den flexiblen Mantel am flexiblen Abschnitt des Lichtleiters gehalten. Als Gleitmittel sind ferner Materialien verwendbar, die als Schlichten bekannt sind.

Die Verwendung eines Gleitmittels innerhalb des Mantels ermöglicht eine deutliche Herabsetzung der Reibung bzw. der Reibungskräfte (Gleitreibung und insbesondere Haftreibung) zwischen den lichtleitenden Fasern sowie zwischen den lichtleitenden Fasern und dem Mantel. Bei reduzierter Reibung sinkt das Risiko eines Bruchs einzelner lichtleitender Fasern deutlich. Dies erhöht die Lebenserwartung des Lichtleiters und des gesamten Endoskops. Die Verwendung des flexiblen Mantels um den flexiblen Abschnitt des Lichtleiters ermöglicht eine Verwendung von Gleitmitteln, die anderenfalls aufgrund ihrer Konsistenz nicht verwendbar wären, weil sie aus dem Bündel von lichtleitenden Fasern heraus fließen oder fallen würden.

Bei einem Endoskop, wie es hier beschrieben ist, bei dem der Lichtleiter ein Bündel aus lichtleitenden Fasern umfasst, können lichtleitende Fasern des Bündels eine Beschichtung zur Herabsetzung von Reibung zwischen den lichtleitenden Fasern aufweisen.

Die einzelnen lichtleitenden Fasern können unabhängig davon, ob das Bündel im Bereich des flexiblen Abschnitts in einem flexiblen Mantel angeordnet ist, eine Beschichtung zur Herabsetzung von Reibung aufweisen. Davon abgesehen ähneln die Vorteile einer Beschichtung der Fasern zur Herabsetzung von Reibung den oben dargestellten Vorteilen der Verwendung eines Gleitmittels.

Bei einem Endoskop, wie es hier beschrieben ist, bei dem der Lichtleiter ein Bündel von lichtleitenden Fasern umfasst, kann das Bündel einen nicht-kreisförmigen Querschnitt aufweisen.

Das Bündel weist insbesondere einen im Wesentlichen elliptischen, ovalen oder rechteckigen Querschnitt auf, wobei die Breite bzw. Höhe des Querschnitts in zwei zueinander senkrechten Richtungen sich mindestens wie 3:2 oder mindestens wie 2:1 oder mindestens wie 5:1 oder mindestens wie 10:1 verhält. Insbesondere weist das Bündel einen Querschnitt auf, dessen Höhe geringer ist als seine Breite, wobei die Höhe in einer Richtung gemessen ist, in der die Krümmung des Lichtleiters bei seiner vorgesehenen elastischen Verformung verändert wird, und wobei die Breite des Bündels in einer dazu senkrechten Richtung gemessen wird.

Ein nicht-kreisförmiger Querschnitt des Bündels, insbesondere ein abgeflachter Querschnitt, wie er oben beschrieben ist, erhöht die Flexibilität des Bündels und reduziert die mechanische Belastung einzelner lichtleitender Fasern bei der vorgesehenen elastischen Verformung des Lichtleiters. Dadurch können das Risiko eines Defekts am Lichtleiter reduziert und die Lebensdauer des Lichtleiters und des gesamten Endoskops erhöht werden.

Ferner wird durch einen nicht-kreisförmigen Querschnitt des Bündels die zur vorgesehenen Verformung des Lichtleiters erforderliche Kraft reduziert. Dadurch können Einrichtungen zum Halten, Führen und Bewegen der Lichtaustrittseinrichtung und zum elastischen Verformen des Lichtleiters kleiner dimensioniert werden. Ferner können die zur Einstellung der Blickrichtung und der Beleuchtungsrichtung sowie zur elastischen Verformung des Lichtleiters erforderlichen Kräfte reduziert werden.

Ein Endoskop, wie es hier beschrieben ist, kann eine Mehrzahl von Lichtleitern umfassen, die jeweils einen flexiblen Abschnitt aufweisen, wobei der flexible Abschnitt jedes Lichtleiters dazu ausgebildet und angeordnet ist, elastisch verformt zu werden, wenn die Lichtaustrittseinrichtung bewegt wird.

Bei gleichem Gesamtquerschnitt und damit gleicher übertragbarer Gesamtstrahlungsleistung weisen mehrere Lichtleiter jeweils einen geringeren Querschnitt auf als ein einziger Lichtleiter. Eine Mehrzahl von Lichtleitern kann deshalb eine höhere Flexibilität aufweisen als ein einziger Lichtleiter. Dies kann das Risiko eines Defekts an einem Lichtleiter reduzieren. Ferner kann bei Ausfall eines Lichtleiters weiterhin Licht über weitere Lichtleiter übertragen werden. Ein Defekt an einem Lichtleiter muss deshalb keinen Totalausfall des Endoskops zur Folge haben.

Die höhere Flexibilität einer Mehrzahl von Lichtleitern mit jeweils kleinerem Querschnitt hat ferner zur Folge, dass die zum Einstellen der Blickrichtung und der Beleuchtungsrichtung und zur vorgesehenen elastischen Verformung der Lichtleiter erforderlichen Kräfte geringer sind.

Insbesondere umfasst das Endoskop zwei Lichtleiter mit flexiblen Abschnitten, wobei den Lichtleitern jeweils zugeordnete Lichtaustrittseinrichtungen an zwei gegenüberliegenden Seiten eines Lichteintrittsfensters eines Beobachtungsstrahlengangs angeordnet sind. Diese Anordnung von mindestens zwei Lichtaustrittseinrichtungen kann eine besonders schattenarme Ausleuchtung des Sichtfelds ermöglichen.

Bei einem Endoskop, wie es hier beschrieben ist, kann die Lichtaustrittseinrichtung um eine Schwenkachse schwenkbar sein.

Eine Schwenkbarkeit der Lichtaustrittseinrichtung um eine Schwenkachse ist insbesondere vorteilhaft, wenn die Blickrichtung des Endoskops um eine Schwenkachse schwenkbar ist. Die Schwenkachse der Lichtaustrittseinrichtung ist in diesem Fall insbesondere die Schwenkachse der Blickrichtung. Die Schwenkachse der Lichtaustrittseinrichtung ist insbesondere senkrecht zur Längsachse des Endoskops bzw. zur Längsachse des Schafts des Endoskops.

Ein Endoskop, wie es hier beschrieben ist, kann ferner ein Gelenk umfassen, um das die Lichtaustrittseinrichtung schwenkbar ist.

Das Gelenk umfasst insbesondere eine Welle, wobei die Achse der Welle die Schwenkachse der Lichtaustrittseinrichtung ist. Eine derartige Ausgestaltung des Gelenks kann eine einfache und gleichzeitig präzise Führung der Lichtaustrittseinrichtung ermöglichen.

Bei einem Endoskop mit einem Gelenk, das eine Welle umfasst, kann die Welle so ausgebildet sein, dass der Lichtleiter beim Schwenken der Lichtaustrittseinrichtung an einem Ende der Welle vorbei bewegbar ist.

Diese Ausgestaltung der Welle ermöglicht einen besonders großen Raum, innerhalb dessen der Lichtleiter bei seiner Verformung sich bewegen kann. Dadurch können die erforderlichen Krümmungen reduziert bzw. die erforderlichen Krümmungsradien erhöht werden. Durch eine reduzierte Krümmung des Lichtleiters können auch bei häufiger Verformung das Risiko eines Defekts des Lichtleiters verringert und die Lebensdauer des Lichtleiters und des Endoskops erhöht werden. Ferner können die zur Einstellung der Blickrichtung und der Beleuchtungsrichtung sowie zur vorgesehenen Verformung des Lichtleiters erforderlichen Kräfte reduziert werden.

Bei einem Endoskop, wie es hier beschrieben ist, kann eine Kulissenführung zum Führen der Lichtaustrittseinrichtung vorgesehen sein.

Die Kulissenführung ist insbesondere bogenförmig oder kreisbogenförmig ausgeführt. Die Kulisseführung stellt insbesondere eine Alternative zu einem Gelenk dar, kann jedoch auch mit einer Welle oder einem anderen Gelenk kombiniert sein. Die Kulissenführung umfasst insbesondere einen Schlitten, der in einer oder mehreren Führungsnuten oder an einem oder mehreren Stegen oder Schienen geführt ist.

Ein Vorteil einer Kulissenführung kann darin bestehen, dass kein Bauraum für eine Welle oder ein anderes Gelenk erforderlich ist. Dadurch kann der für den oder die Lichtleiter und für deren Bewegung bereitstehende Raum besonders groß sein.

Ein Endoskop mit einer Kulissenführung, wie es hier beschrieben ist, kann ferner eine Kraftübertragungseinrichtung zum Übertragen einer Schub- oder Zugkraft zum proximalen Ende des Endoskops zum Bewegen der Lichtaustrittseinrichtung aufweisen, wobei die Kraftübertragungseinrichtung mit einem Schlitten der Kulissenführung einstückig ausgebildet ist.

Die Kraftübertragungseinrichtung erstreckt sich insbesondere vom distalen Ende des Endoskops bis zum proximalem Ende des Endoskops, um die Schub- oder Zugkraft vom proximalen zum distalen Ende des Endoskops zu übertragen. Die Kraftübertragungseinrichtung kann am proximalen Ende des Endoskops mit einer Handhabungseinrichtung zum Bewegen der Lichtaustrittseinrichtung mittels der Kraftübertragungseinrichtung gekoppelt sein.

Die Kraftübertragungseinrichtung umfasst beispielsweise eine Schubstange, ein Zugeinrichtung, eine Kuppelstange oder eine andere, insbesondere stab- oder rohrförmige Einrichtung. Die Kraftübertragungseinrichtung kann so biegesteif ausgeführt sein, dass sie bei einer vorgesehenen maximalen Schub- bzw. Druckbelastung nicht knickt. Alternativ oder zusätzlich kann die Kraftübertragungseinrichtung an einer oder mehreren Stellen oder in einem oder mehreren Abschnitten geführt sein, um ein Knicken bei Schub- bzw. Druckbelastung zu verhindern.

Ein die Kraftübertragungseinrichtung und den Schlitten bildendes Bauteil kann zumindest im Bereich der Kulissenführung dünn und biegeflexibel ausgeführt sein, um sich einer Bogenform der Führungsnuten, Stege bzw. Schienen anpassen zu können. Wenn die Kraftübertragungseinrichtung beispielsweise aufgrund einer Federbelastung am distalen Ende ausschließlich oder überwiegend zur Übertragung von Zugkräften vorgesehen ist, kann das die Kraftübertragungseinrichtung und den Schlitten bildende Bauteil über seine gesamte Länge flexibel ausgebildet sein. Gleiches gilt, wenn die Kraftübertragungseinrichtung ausreichend geführt ist, um auch im Fall einer Schub- bzw. Druckbelastung ein Knicken zu verhindern. In diesem Fall kann das die Kraftübertragungseinrichtung und den Schlitten bildende Bauteil beispielsweise ein dünner und biegeflexibler Blechstreifen sein.

Wenn die Kraftübertragungseinrichtung zur Übertragung einer mehr als geringfügigen Schub- bzw. Druckkraft vorgesehen ist, kann das die Kraftübertragungseinrichtung und den Schlitten bildende Bauteil im Bereich der Kraftübertragungseinrichtung biegesteif ausgebildet und/oder geführt sein, um ein Knicken zu verhindern. Im Fall einer Ausführung des die Kraftübertragungseinrichtung und den Schlitten bildenden Bauteils als biegeflexibler Blechstreifen ist die Biegesteifigkeit des Blechstreifens im Bereich der Kraftübertragungseinrichtung beispielsweise durch eine oder mehrere Längssicken oder zu Längsflanschen umgebogene Ränder erhöht.

Bei einem Endoskop mit einer Kraftübertragungseinrichtung, wie es hier beschrieben ist, kann der Lichtleiter an der Kraftübertragungseinrichtung mechanisch geführt sein.

Insbesondere ist der Lichtleiter an zumindest einem Ort mit der Kraftübertragungseinrichtung mechanisch starr verbunden. Alternativ oder zusätzlich kann der Lichtleiter an einer oder mehreren Stellen an der Kraftübertragungseinrichtung in seiner Längsrichtung geführt sein. Eine Führung des Lichtleiters in seiner Längsrichtung bedeutet, dass der Lichtleiter sich in seiner Längsrichtung bewegen kann, nicht jedoch in den beiden Richtungen senkrecht zur Längsrichtung des Lichtleiters. Die Längsrichtung des Lichtleiters entspricht insbesondere der Längsrichtung der Kraftübertragungseinrichtung.

Bei einem Endoskop, wie es hier beschrieben ist, liegen ein Mittelpunkt der Lichtaustrittseinrichtung und ein Mittelpunkt der Lichteintrittsfläche des Beobachtungsstrahlengangs insbesondere in einer Ebene parallel zu einer Schwenkachse der Blickrichtung oder in einer Ebene senkrecht zur Schwenkachse der Blickrichtung.

Im ersten Fall liegt die Lichtaustrittseinrichtung bezogen auf die Schwenkrichtung der Beleuchtungsrichtung und der Blickrichtung neben der Lichteintrittsfläche des Beobachtungsstrahlengangs. Im zweiten Fall liegen die Lichtaustrittseinrichtung und die Lichteintrittsfläche des Beobachtungsstrahlengangs bezogen auf die Schwenkrichtung der Beleuchtungsrichtung und der Blickrichtung hinter einander. Zur Minderung von Schattenwurf können in beiden Fällen jeweils zwei oder mehr Lichtaustrittseinrichtungen an mindestens zwei gegenüberliegenden Seiten der Lichtseintrittsfläche des Beobachtungsstrahlengangs angeordnet sein.

Bei einem Endoskop, wie es hier beschrieben ist, können eine Einrichtung zum Einstellen der Blickrichtung und die Lichtaustrittseinrichtung unmittelbar oder mittelbar am distalen Ende des Endoskops mechanisch gekoppelt sein, um eine synchrone Bewegung der Blickrichtung und der Beleuchtungsrichtung zu ermöglichen.

Die Einrichtung zum Einstellen der Blickrichtung umfasst beispielsweise eine Einrichtung zum Schwenken einer Kamera bzw. eines lichtempfindlichen Sensors, eines Prismas, eines Spiegels oder eines oder mehrerer anderer optischer Elemente im Beobachtungsstrahlengang des Endoskops. Zur unmittelbaren Kopplung der Einrichtung zum Einstellen der Blickrichtung und der Lichtaustrittseinrichtung sind diese beispielsweise an einer gemeinsamen Welle oder an einer gemeinsamen Kulissenführung angeordnet. Die gemeinsame Kulissenführung oder die gemeinsame Welle ist beispielsweise mittels der oben erwähnten Kraftübertragungseinrichtung betätigbar bzw. bewegbar.

Eine mittelbare Kopplung kann erforderlich sein, wenn ein oder mehrere optische Elemente im Beobachtungsstrahlengang und die Lichtaustrittseinrichtung um unterschiedliche Winkel (beispielsweise im Verhältnis 1:2) bewegt werden müssen. Eine mittelbare Kopplung ist beispielsweise am proximalen Ende möglich, an dem eine Handhabungseinrichtung zum Einstellen der Blickrichtung vorgesehen ist. Eine manuelle Einwirkung auf die Handhabungseinrichtung kann beispielsweise über zwei getrennte Wellen, Schubstangen, Zugelemente oder Kuppelstangen einerseits zum Schwenken der Beleuchtungsrichtung durch eine Bewegung der Lichtaustrittseinrichtung und andererseits zum Schwenken der Blickrichtung an das distale Ende des Endoskops übertragen werden.

Für fluiddichte Durchführungen können erforderlichenfalls Magnetkupplungen, Faltenbälge oder Dichtungen verwendet werden, die proximal und/oder distal angeordnet sein können.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung einer Ausführungsform des distalen Endes des Endoskops aus Figur 1;
- Figur 3: eine weitere schematische Darstellung der Ausführungsform der Figur 2;
- Figur 4: eine weitere schematische Darstellung der Ausführungsform der Figuren 2 und 3;
- Figur 5: eine weitere schematische Darstellung der Ausführungsform der Figuren 2 bis 4;
- Figur 6: eines schematische Darstellung einer weiteren Ausführungsform des distalen Endes des Endoskops die nicht teil der Erfindung ist;
- Figur 7: eine weitere schematische Darstellung der Ausführungsform der Figur 6;
- Figur 8: eine weitere schematische Darstellung der Ausführungsform der Figuren 6 und 7;
- Figur 9: eine schematische Darstellung eines Querschnitts eines Lichtleiters;
- Figur 10: eine schematische Darstellung einer weiteren Ausführungsform des distalen Endes des Endoskops aus Figur 1;
- Figur 11: eine weitere schematische Darstellung der Ausführungsform der Figur 10;
- Figur 12: eine schematische Darstellung einer weiteren Ausführungsform des distalen Endes des Endoskops aus Figur 1;
- Figur 13: eine weitere schematische Darstellung der Ausführungsform der Figur 12;
- Figur 14: eine schematische Darstellung einer weiteren Ausführungsform des distalen Endes des Endoskops aus Figur 1;
- Figur 15: eine weitere schematische Darstellung der Ausführungsform der Figur 14.

### Beschreibung von Ausführungsformen

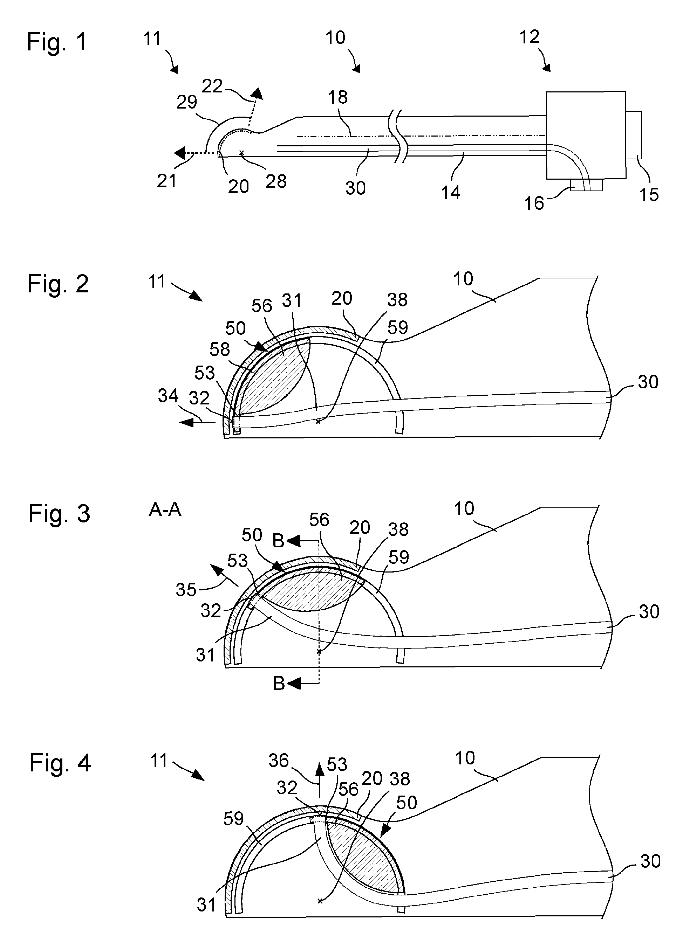

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellt Längsachse 18 des Endoskops 10 die Symmetrieachse der Mantelfläche des Schafts 14 zwischen dem distalen Ende 12 und dem proximalen Ende 11. Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 11. Bei einer kreiszylindrischen Mantelfläche des Schafts 14 ist die Längsachse 18 auch die Symmetrieachse der Mantelfläche.

Am distalen Ende 12 weicht die Gestalt des Schafts 14 von der Zylindersymmetrie ab, wie dies beispielhaft in Figur 1 dargestellt ist. Insbesondere weist der Schaft 14 am distalen Ende 12 eine Öffnung auf, die durch ein transparentes Fensterbauteil 20 mit einer gewölbten Oberfläche verschlossen ist. Insbesondere verschließt das Fensterbauteil 20 die Öffnung hermetisch dicht. Die Oberfläche des Fensterbauteils 20 hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche des transparenten Fensterbauteils 20 die Gestalt eines Ausschnitts einer Kugeloberfläche oder eines rotationssymmetrischen oder nicht rotationssymmetrischen Ellipsoids.

Am distalen Enden 12 des Endoskops 10 sind im Schaft 14 optische Einrichtungen angeordnet, die in Figur 1 nicht dargestellt sind. Diese optischen Einrichtungen ermöglichen eine Variation der Blickrichtung des Endoskops zwischen einer ersten extremen Blickrichtung 21 und einer zweiten extremen Blickrichtung 22. Die Blickrichtung ist zwischen den beiden extremen Blickrichtungen 21, 22 um eine Schwenkachse 28 schwenkbar, die senkrecht zur Zeichenebene der Figur 1 ist. Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste extreme Blickrichtung 21 parallel oder im Wesentlichen parallel zur Längsachse 18 des Endoskops 10. Zwischen den extremen Blickrichtungen 21, 21 liegt ein Winkelbereich 29, der bei dem dargestellten Beispiel ca. 120 Grad umfasst. Innerhalb dieses Winkelbereichs ist die Blickrichtung des Endoskops 10 insbesondere kontinuierlich verstellbar bzw. einstellbar.

Am proximalen Ende 11 weist das Endoskop 10 eine erste Kupplung 15 zum optischen Koppeln des Endoskops 10 mit einer Kamera oder ein Okular sowie eine zweite Kupplung 16 zum Koppeln des Endoskops 10 mit einer Lichtquelle über ein Lichtleitkabel auf. Von der zweiten Kupplung 16 führen ein oder mehrere Lichtleiter 30 durch den Schaft 14 bis zum distalen Ende 11 des Endoskops 10. Von einer Lichtquelle erzeugtes Beleuchtungslicht kann über ein Lichtleitkabel, die zweite Kupplung 16 und den oder die Lichtleiter 30 zum distalen Ende 11 des Endoskops 10 übertragen werden.

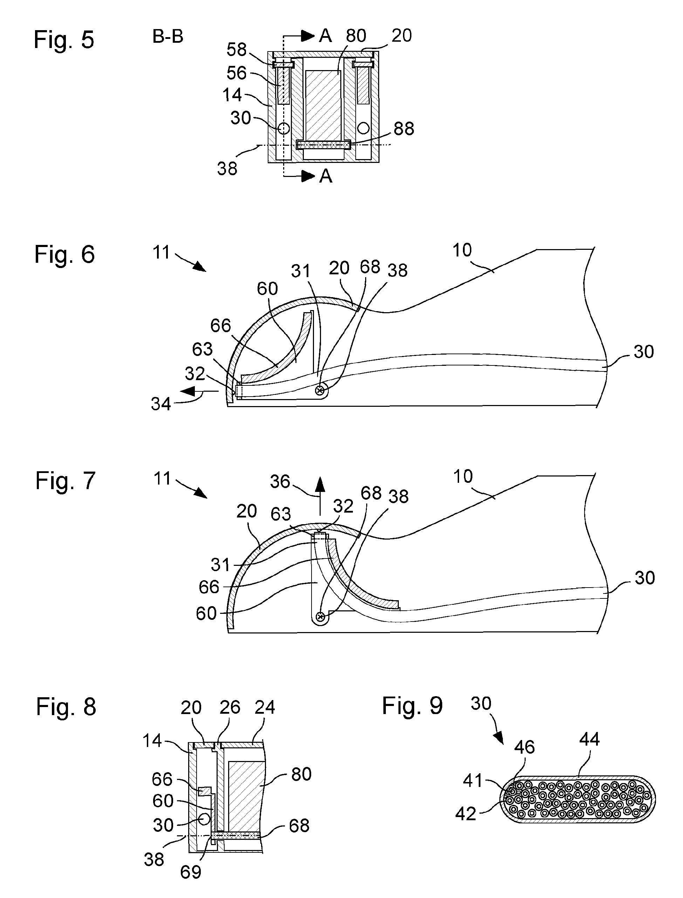

Die Figuren 2 bis 5 zeigen schematische Darstellungen einer Ausführungsform des distalen Endes 11 des oben anhand der Figur 1 dargestellten Endoskops 10. Die Figuren 2 bis 4 zeigen einander entsprechende Darstellungen von Schnitten entlang einer Ebene A-A parallel zur Zeichenebene der Figur 1. Die Lage der Schnittebene A-A ist in Figur 5 angedeutet. Figur 5 zeigt eine schematische Darstellung eines Schnitts entlang einer Schnittebene B-B senkrecht zu den Zeichenebenen bzw. Schnittebenen der Figuren 1 bis 4. Ein Teil der nachfolgend beschriebenen Teile des Endoskops sind bei den Darstellungen der Figuren 2 bis 4 unterschiedlich angeordnet. Hinsichtlich der Anordnung dieser Elemente entsprechen einander nur die Figuren 3 und 5. Die näherungsweise quadratische äußere Kontur des in Figur 5 dargestellten Querschnitts stellt eine Vereinfachung dar, tatsächlich kann der Querschnitt davon abweichen, beispielsweise mit einer zumindest teilweise kreisbogenförmigen äußeren Kontur.

Der Lichtwellenleiter 30 weist am distalen Ende 11 des Endoskops 10 einen flexiblen Abschnitt 31 und eine Lichtaustrittsfläche 32 auf. Der Lichtleiter 30 umfasst beispielsweise ein Bündel von lichtleitenden Fasern, deren Enden in einer Ebene abgeschnitten und poliert sind, um die Lichtaustrittsfläche 32 zu bilden. Die Lichtaustrittsfläche 32 stellt eine einfache Form einer Lichtaustrittseinrichtung dar. Eine Lichtaustrittseinrichtung kann abweichend von der Darstellung in den Figuren 2 bis 4 ferner eine oder mehrere Linsen, Prismen, Spiegel oder andere optische Elemente zur Strahlformung aufweisen.

Das Endoskop 10 weist am distalen Ende 11 eine Führungseinrichtung 50 zum Führen der Lichtaustrittsfläche 32 des Lichtleiters 30 auf. Die Führungseinrichtung 50 umfasst einen Schlitten 58 mit einer Fassung 53 für den Lichtleiter 30. Die Fassung 53 umfasst beispielsweise eine Durchgangsbohrung, in die der Lichtleiter 30 nahe der Lichtaustrittsfläche 32 beispielsweise mittels Klebstoff gefügt ist.

Der Schlitten 58 weist bei dem in den Figuren 2 bis 5 dargestellten Beispiel im Wesentlichen die Gestalt einer gekrümmten Platte auf, die einen Teil eines Kreiszylindermantels bildet. Zwei gegenüberliegende, jeweils kreisbogenförmige Kanten des Schlittens 58 sind in Führungsnuten 59 im Schaft 14 des Endoskops bzw. in dem den Schaft 14 bildenden Gehäuse geführt. Die Führungsnut 59 ist kreisbogenförmig. Ferner weist die Führungseinrichtung 50 eine Anlageeinrichtung 56 auf.

In den Figuren 2 bis 4 sind der Schlitten 58 sowie die Anlageeinrichtung 56 und die Lichtaustrittsfläche 32 des Lichtleiters 30, die mit dem Schlitten 58 starr verbunden sind, in drei verschiedenen Positionen dargestellt. Der Schlitten 58, die Lichtaustrittsfläche 32 und die Anlageeinrichtung 56 können zwischen den in den Figuren 2 bis 4 dargestellten Positionen bewegt werden. Die Führung des Schlittens 58 in den kreisbogenförmigen Nuten 59 zwingt den Schlitten 58 die Lichtaustrittsfläche 32 und die Anlageeinrichtung 56 dabei auf kreisbogenförmige Bahnen um eine Schwenkachse 38. Geometrische Mittelpunkte der kreisbogenförmigen Führungsnuten 59 liegen auf der Schwenkachse 38. Bei den in den Figuren 2 bis 4 gezeigten Positionen der Lichtaustrittsfläche 32 wird vom Lichtleiter 30 übertragenes Beleuchtungslicht gegebenenfalls in die erste Beleuchtungsrichtung 34 (Figur 2), in die zweite Beleuchtungsrichtung 35 (Figur 3) bzw. in die dritte Beleuchtungsrichtung 36 (Figur 4) ausgekoppelt bzw. abgestrahlt.

Bei einem Verfahren bzw. Bewegen der Lichtaustrittsfläche 32 mit dem Schlitten 58 und der Anlageeinrichtung 56 zwischen den in den Figuren 2 bis 4 gezeigten Positionen wird der elastische Abschnitt 31 des Lichtleiters 30 elastisch verformt. Bei der in Figur 2 gezeigten ersten Beleuchtungsrichtung 34 verläuft der Lichtleiter 30 im Wesentlichen gerade. Bei der in Figur 3 gezeigten zweiten Beleuchtungsrichtung 35 ist der elastische Abschnitt 31 des Lichtleiters 30 leicht gekrümmt. Bei der in Figur 4 gezeigten dritten Beleuchtungsrichtung 36 ist der elastische Abschnitt 31 des Lichtleiters 30 noch stärker gekrümmt als bei der in Figur 3 gezeigten zweiten Beleuchtungsrichtung 35.

Eine kreisbogenförmige Anlagefläche der Anlageeinrichtung 56 bewirkt eine gleichmäßige Krümmung des elastischen Abschnitts 31 des Lichtleiters 30 insbesondere bei der dritten Beleuchtungsrichtung 36 und verhindert ein Knicken bzw. eine ungleichförmige Krümmung des elastischen Abschnitts 31 des Lichtleiters 30.

Insbesondere in Figur 5 ist erkennbar, dass dem Lichtleiter 30 viel Raum zur Verfügung steht. Dies ermöglicht auch bei extremen Blickrichtungen 21, 22 (vgl. Figur 1) eine vergleichsweise schwache Krümmung des elastischen Abschnitts 31 des Lichtleiters 30 und senkt so das Risiko eines Defekts.

In Figur 5 ist ferner eine Kamera 80 erkennbar, die an einer Welle 88 um die Schwenkachse 38 der Beleuchtungsrichtung schwenkbar befestigt ist. Durch die Schwenkbarkeit der Kamera 80 weist das Endoskop 10 eine schwenkbare Blickrichtung auf, wie dies oben anhand der Figur 1 dargestellt wurde. Abweichend von der Darstellung in Figur 5, bei der die Schwenkachse 38 der Beleuchtungsrichtung der oben anhand der Figur 1 dargestellten Schwenkachse 28 der Blickrichtung entspricht, kann die Kamera 80 um eine zur Schwenkachse 38 der Beleuchtungsrichtung parallele Schwenkachse geschwenkt werden. Die Schwenkachse 28 der Blickrichtung 21, 22 und die Schwenkachse 38 der Beleuchtungsrichtung 34, 35, 36 müssen also nicht identisch sein. Eine Parallelität beider Schwenkachsen 28, 38 ist aber in vielen Fällen vorteilhaft. Anstelle einer Kamera 80 können ferner ein oder mehrere Prismen, Spiegel oder andere optische Elemente zum Einstellen der Blickrichtung vorgehen sein.

Die Figuren 6 bis 8 zeigen schematische Darstellungen einer weiteren Ausführungsform des distalen Endes 11 des oben anhand der Figur 1 dargestellten Endoskops 10. Die Figuren 6 und 7 zeigen schematische Darstellungen eines Schnitts entlang einer Ebene parallel zur Zeichenebene der Figur 1 und entsprechend den Zeichenebenen der Figuren 2 bis 4. Figur 8 zeigt eine schematische Darstellung eines Schnitts entlang einer Ebene senkrecht zu den Zeichenebenen bzw. Schnittebenen der Figuren 1, 6 und 7. Da die Darstellung in Figur 8 hinsichtlich der Position von nachfolgend beschriebenen Einrichtungen keiner der Figuren 6 und 7 entspricht, sind die Schnittebenen bzw. ihre Lagen in den Figuren 6 bis 8 nicht benannt bzw. angedeutet.

Die Ausführungsform der Figuren 6 bis 8 ähnelt der oben anhand der Figuren 2 bis 5 dargestellten Ausführungsform in einigen Merkmalen. Die Ausführungsform der Figuren 6 bis 8 unterscheidet sich von der oben anhand der Figuren 2 bis 5 dargestellten Ausführungsform insbesondere dadurch, dass zur Führung der Lichtaustrittsfläche 32 des Lichtleiters 30 keine Schlitten und keine Führungsnut vorgesehen sind. Stattdessen ist eine Führungseinrichtung 60 mit einer Fassung 63 zum Halten des Lichtleiters 30 nahe dessen Lichtaustrittsfläche 32 vorgesehen. Die Führungseinrichtung 60 weist eine Anlageeinrichtung 66 auf, die in Form und Funktion der Anlageeinrichtung bei der Ausführungsform der Figuren 2 bis 5 ähnelt. Im Unterschied zur Ausführungsform der Figuren 2 bis 5 ist die Führungseinrichtung 60 an einer Welle 68 befestigt und mit dieser um die Schwenkachse 38 der Beleuchtungsrichtung schwenkbar.

In den Figuren 6 und 7 sind die Führungseinrichtung 60 und die Lichtaustrittsfläche 32 bei der ersten Beleuchtungsrichtung 34 (vgl. Figur 2) bzw. bei der dritten Beleuchtungsrichtung 36 (vgl. Figur 4) gezeigt. Die Beleuchtungsrichtung und die Lichtaustrittsfläche 32 sind mit der Führungseinrichtung 60 um die Schwenkachse 38 zwischen den in den Figuren 6 und 7 gezeigten Positionen frei schwenkbar, um beliebige Beleuchtungsrichtungen zumindest zwischen der ersten Beleuchtungsrichtung 34 und der dritten Beleuchtungsrichtung 36 einzustellen.

Insbesondere in dem in Figur 8 dargestellten Querschnitt ist erkennbar, dass dem Lichtleiter 30 beim Schwenken der Lichtaustrittsfläche 32 um die Schwenkachse 38 ein großer Raum zur Verfügung steht. Der dem Lichtleiter 30 zur Verfügung stehende Raum ist insbesondere aufgrund der Position des Endes 69 der Welle 68 nicht durch die Welle 68 eingeschränkt. Deshalb kann der Lichtleiter 30 bei der ersten Beleuchtungsrichtung 34 abweichend von der Darstellung in Figur 6 noch deutlich gerader verlaufen. Die Anlageeinrichtung 66 gewährleistet insbesondere bei der dritten Beleuchtungsrichtung 69 eine gleichmäßige Krümmung des elastischen Abschnitts 31 des Lichtleiters 30.

In Figur 8 ist ferner erkennbar, dass eine Kamera 80 an der Welle 68 der Führungseinrichtung angeordnet ist. Durch eine Rotation der Welle 68 werden somit gleichzeitig die Lichtaustrittsfläche 32 des Lichtleiters 30, die Beleuchtungsrichtung und die Kamera 80 und damit auch die Blickrichtung geschwenkt. Die Welle 68 koppelt somit unmittelbar die Führungseinrichtung 60 bzw. die mit der Führungseinrichtung 60 starr verbundene Lichtaustrittsfläche 32 einerseits und die Kamera 80 andererseits. Damit koppelt die Welle 68 auch deren Bewegungen.

Bei der Ausführungsform der Figuren 2 bis 5 ist ein durchgehendes bzw. gemeinsames Fensterbauteil 20 vorgesehen, durch das sowohl Beleuchtungslicht aus dem distalen Ende 11 des Endoskops 10 austreten als auch von einem beobachteten Gegenstand ausgehendes Licht in das distale Ende 11 des Endoskops 10 eintreten kann. Dies ist insbesondere in Figur 5 erkennbar. Der Raum, in dem sich Beleuchtungslicht ausbreiten kann, ist lediglich bis zur inneren Oberfläche des Fensterbauteils 20 von dem Raum, in dem die Kamera 80 angeordnet ist, durch eine Wand getrennt bzw. optisch isoliert.

Bei der Ausführungsform der Figuren 6 bis 8 sind mehrere separate Fensterbauteile vorgesehen. Ein mit dem Bezugszeichen 20 versehenes Fensterbauteil ist für den Austritt von Beleuchtungslicht aus dem distalen Ende 11 des Endoskops 10 vorgesehen. Ein weiteres Fensterbauteil 24 ist für den Eintritt von Licht, das von einem beobachteten Gegenstand ausgeht, in das distale Ende 11 des Endoskops 10 vorgesehen. Beide Fensterbauteile 20, 24 sind durch eine Trennwand 26 bzw. deren Randbereich getrennt, die nicht lichtdurchlässig ist. Die Trennwand 26 ist insbesondere ausgebildet, um den Raumbereich, in dem Beleuchtungslicht sich ausbreitet, von dem Raumbereich, in dem die Kamera 80 angeordnet ist, bis zur äußeren Oberfläche des Endoskops 10 vollständig und vor allem lichtdicht voneinander zu trennen. Dadurch kann eine Einkopplung von Beleuchtungslicht, das im Fensterbauteil 24 oder an seiner Oberfläche gestreut wird, in die Kamera 80 verhindert werden.

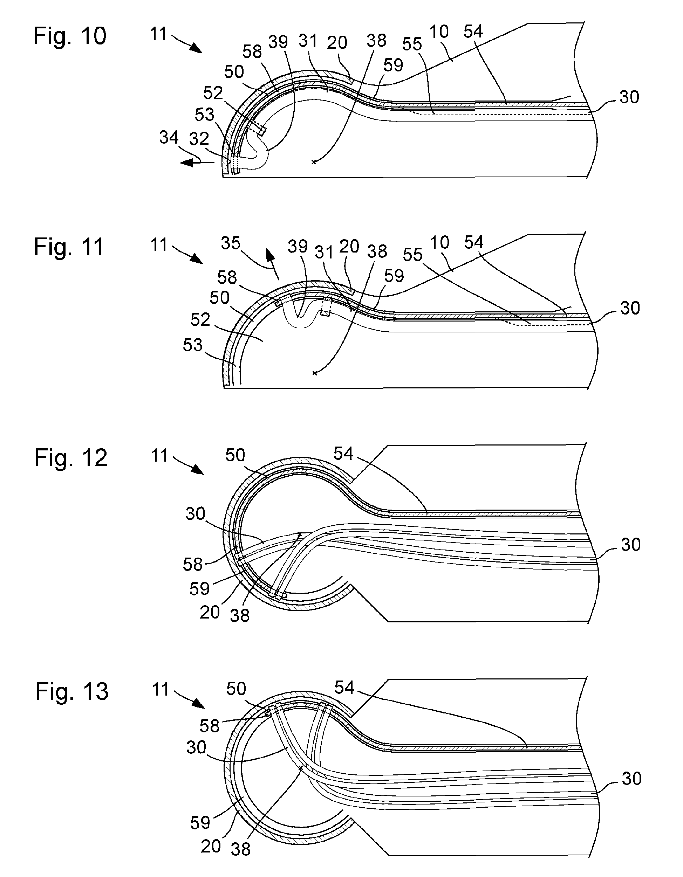

Bei jeder der hier beschriebenen Ausführungsformen, also sowohl bei den Ausführungsformen der Figuren 2 bis 5 und 6 bis 8 als auch bei nachfolgend anhand der Figuren 10 bis 15 dargestellten Ausführungsformen, können ein durchgehendes Fensterbauteil oder mehrere separate Fensterbauteile vorgesehen sein. Ferner kann ein durchgehendes Fensterbauteil, wie es in Figur 5 erkennbar ist, mit einer Licht absorbierenden Trennschicht versehen sein, die eine unerwünschte unmittelbare Einkopplung von Beleuchtungslicht in die Kamera 80 verhindert. Eine solche Trennschicht kann beispielsweise durch implantierte oder auf andere Weise in das Material des Fensterbauteils lokal eingebrachte Ionen erzeugt werden.

Insbesondere in den Figuren 5 und 8 sind kreisförmige Querschnitte des Lichtleiters 30 gezeigt. Figur 9 zeigt einen alternativen Querschnitt des Lichtleiters 30, der bei beiden oben anhand der Figuren 2 bis 8 dargestellten Ausführungsformen verwendbar ist. Der Lichtleiter umfasst ein flaches Bündel von lichtleitenden Fasern 41, die jeweils eine Beschichtung 42 zur Herabsetzung der Reibung aufweisen. Das Bündel von lichtleitenden Fasern 41 ist in einem flexiblen Mantel 44 angeordnet. Der flexible Mantel 44 umschließt das Bündel lichtleitender Fasern 41 des Lichtleiters 30 insbesondere in dessen elastischen Abschnitt 31 und reicht bis zur Fassung 53, 63 der Führungseinrichtung 50, 60 (vgl. Figuren 2 bis 4, 6, 7).

Talkum oder ein anderes pulverförmiges, flüssiges oder sehr duktiles Gleitmittel 46 kann innerhalb des Mantels 44 zwischen den lichtleitenden Fasern 41 angeordnet sein, um Reibung zwischen den lichtleitenden Fasern 41 sowie zwischen den lichtleitenden Fasern 41 und dem Mantel 44 zu verringern. Der Mantel 44 verhindert ein Herausfallen bzw. Herausfließen des Gleitmittels 46 aus dem Bündel lichtleitender Fasern 41 und kann so zu dessen langfristiger Wirkung beitragen.

Abweichend von der Darstellung in Figur 9 kann der Lichtleiter 30 entweder lediglich Beschichtungen 42 an den lichtleitenden Fasern 41 oder nur ein Gleitmittel 46 zwischen unbeschichteten lichtleitenden Fasern 41 aufweisen.

Figur 9 zeigt einen ovalen Querschnitt, dessen Rand zwei kreisbogenförmige und zwei gerade Abschnitte aufweist. Abweichend von der Darstellung in Figur 9 kann der Lichtleiter einen elliptischen, kreisförmigen oder beliebigen anderen Querschnitt aufweisen.

Die Figuren 10 und 11 zeigen schematische Darstellungen einer weiteren Ausführungsform des distalen Endes 11 des oben anhand der Figur 1 dargestellten Endoskops 10 in Schnitten entlang einer Ebene parallel zur Zeichenebene der Figur 1 und entsprechend den Zeichenebenen der Figuren 2 bis 4, 6 und 7. Das distale Ende 11 des Endoskops 10 ist in den Figuren 10 und 11 bei zwei verschiedenen Beleuchtungsrichtungen 34, 35 dargestellt.

Die Ausführungsform der Figuren 10 und 11 ähnelt den oben anhand der Figuren 2 bis 8 dargestellten Ausführungsformen in einigen Merkmalen. Insbesondere umfasst die Ausführungsform der Figuren 10 und 11 einen Schlitten, der dem oben anhand der Figuren 2 bis 5 dargestellten Schlitten ähnelt. Im Unterschied zu der Ausführungsform der Figuren 2 bis 5 ist der Schlitten 58 einstückig mit einer Kraftübertragungseinrichtung 54 ausgeführt. Die Kraftübertragungseinrichtung 54 erstreckt sich vom proximalen Ende 12 bis zum distalen Ende 11 des Endoskops 10. Am proximalen Ende 12 des Endoskops 10 ist die Kraftübertragungseinrichtung 54 insbesondere mit einer Handhabungseinrichtung mechanisch und/oder magnetisch gekoppelt, mittels derer die Kraftübertragungseinrichtung längs des Schafts 14 des Endoskops 10 verschoben werden kann, um die Beleuchtungsrichtung 34, 35 einzustellen.

Der Schlitten 58 und die Kraftübertragungseinrichtung 54 sind insbesondere durch einen biegeflexiblen Blechstreifen gebildet, der sich vom proximalen Ende 12 bis zum distalen Ende 11 des Endoskops 10 erstreckt. Zwei parallele Ränder des Blechstreifens sind am und nahe dem distalen Ende 11 des Endoskops 10 in zwei einander gegenüberliegenden Führungsnuten 59 geführt, von denen in den Figuren 10 und 11 nur eine dargestellt ist. Ein distaler Abschnitt der Führungsnut 59 verläuft im Wesentlichen kreisbogenförmig parallel zum gekrümmten Fensterbauteil 20. Der Mittelpunkt des von der Führungsnut beschriebenen Kreisbogens liegt auf der Schwenkachse 38 der Beleuchtungsrichtung 34, 35.

Der Blechstreifen, der den Schlitten 58 und die Kraftübertragungseinrichtung 54 bildet, weist in dem Bereich, in dem der Blechstreifen die Kraftübertragungseinrichtung 54 bildet, eine Längssicke 55 auf. Die Längssicke 55 versteift den an sich biegeflexiblen Blechstreifen so weit, dass die Kraftübertragungseinrichtung auch bei einer vorbestimmten Druck- bzw. Schubbelastung eine gerade Gestalt beibehält und nicht knickt. Die proximalen Enden der Führungsnuten 59 ist so angeordnet und die Längssicke 55 erstreckt sich so weit nach distal, dass die Führungsnut 59 und die Längssicke 55 bei jeder Blickrichtung 34, 35 überlappen oder zumindest nur wenig voneinander beabstandet sind, und dass die Längssicke 55 bei jeder Blickrichtung 34, 35 an einem gerade Abschnitt der Führungsnut 59 liegt. Dadurch ist sichergestellt, dass einerseits der die Kraftübertragungseinrichtung 54 bildende Abschnitt des Blechstreifens jederzeit eine Druck- bzw. Schubkraft aufnehmen kann ohne zu knicken, und dass andererseits der den Schlitten 58 bildende Abschnitt des Blechstreifens widerstandsarm im gekrümmten Abschnitt der Führungsnut 59 verformt und hin- und herbewegt werden kann.

Wenn die Führungsnut 59 bis zum proximalen Ende 12 des Endoskops 10 reicht, kann der den Schlitten 58 und die Kraftübertragungseinrichtung 54 bildende Blechstreifen auch ohne Längssicke 55 bei Druck- bzw. Schubbelastung nicht knicken. Die Längssicke 55 kann ferner entfallen, wenn die Kraftübertragungseinrichtung 54 nur Zugkräfte oder nur unwesentliche Druck- bzw. Schubkräfte aufnehmen muss. Dazu ist beispielsweise am distalen Ende 11 des Endoskops 10 eine Feder oder ein anderes elastisches Bauteil vorgesehen, das den Schlitten 58 und mit ihm die Kraftübertragungseinrichtung 54 ständig in Richtung zu der Position, die der in Figur 10 dargestellten Beleuchtungsrichtung 34 entspricht, zieht.

Das distale Ende des Lichtleiters 30 ist nahe der Lichtaustrittsfläche 32 des Lichtleiters 30 durch eine Fassung 53 starr am Schlitten 58 gehalten. Ferner ist der Lichtleiter nahe seinem distalen Ende durch eine Schelle 52 oder eine ähnlich wirkende Einrichtung am Schlitten 58 gehalten. Zwischen der Schelle 52 und der Fassung 53 weist der Lichtleiter 30 einen stark gekrümmten Abschnitt 39 auf. Im stark gekrümmten Abschnitt 39 ist der Lichtleiter 30 starr ausgeführt und verformt sich bei Veränderung der Blickrichtung 34, 35 nicht. Wenn der Lichtleiter 39 ein Bündel von Lichtwellenleitern aufweist, sind diese im stark gekrümmten Abschnitt 39 beispielsweise verklebt oder verschmolzen. Anstelle der dargestellten Krümmung kann der Lichtleiter im Abschnitts 39 auf andere Weise gekrümmt sein. Eine schwächere Krümmung ist bei höherem Raumbedarf realisierbar. Anstelle des stark gekrümmten Abschnitts 39 können beispielsweise ein oder mehrere Prismen vorgesehen sein.

Proximal der Schelle 52 ist der Lichtleiter 30 beispielsweise in einem flexiblen Schlauch, der an dem den Schlitten 58 und die Kraftübertragungseinrichtung 54 bildenden Blechstreifen befestigt ist, geführt. Durch den flexiblen Schlauch ist der Lichtleiter 30 in seiner Längsrichtung geführt, d. h. der Lichtleiter 30 kann sich in seiner Längsrichtung bewegen, nicht jedoch in den Richtungen quer dazu. Durch diese Anordnung kann eine definierte Verformung des Lichtleiters 30 mit maximalen Krümmungsradien bzw. minimaler Krümmung verwirklicht werden. Anstelle der Längssicke 55 kann der an der Kraftübertragungseinrichtung 54 geführte Lichtleiter 30 im entsprechenden Abschnitt biegesteif ausgeführt sein, beispielsweise durch Verkleben oder Verschmelzen von einzelnen Lichtwellenleitern, die den Lichtleiter 30 bilden. Alternativ ist die Kraftübertragungseinrichtung 54 im entsprechenden Abschnitt beispielsweise rohrförmig ausgebildet, wobei der Lichtleiter in der rohrförmigen Kraftübertragungseinrichtung 54 geführt ist.

Die Figuren 12 und 13 zeigen schematische Darstellungen einer weiteren Ausführungsform des distalen Endes 11 des oben anhand der Figur 1 dargestellten Endoskops 10 in Schnitten entlang einer Ebene parallel zur Zeichenebene der Figur 1 und entsprechend den Zeichenebenen der Figuren 2 bis 4, 6, 7, 10 und 11. Das distale Ende 11 des Endoskops 10 ist in den Figuren 12 und 13 bei zwei verschiedenen Beleuchtungsrichtungen dargestellt.

Die Ausführungsform der Figuren 12 und 13 ähnelt der oben anhand der Figuren 10 und 11 dargestellten Ausführungsform in einigen Merkmalen. Insbesondere umfasst die Ausführungsform der Figuren 12 und 13 einen Schlitten 58, der mit einer Kraftübertragungseinrichtung 54 einstückig ausgeführt ist. Im Gegensatz zu der Ausführungsform der Figuren 10 und 11 erstrecken sich bei der Ausführungsform der Figuren 12 und 13 die Führungsnuten 59 entlang des gesamten Schafts 14 des Endoskops. Diese Option wurde bereits oben mit Bezug auf die Figuren 10 und 11 beschrieben. Durch die vollständige Führung der Kraftübertragungseinrichtung 54 in durchgehenden Führungsnuten kann die Kraftübertragungseinrichtung 54 auch ohne Längssicke Druck- bzw. Schubkräfte aufnehmen ohne zu knicken.

Ein weiterer Unterschied zur Ausführungsform der Figuren 10 und 11 besteht darin, dass bei der Ausführungsform der Figuren 12 und 13 anstelle eines Lichtleiters mehrere Lichtleiter 30 vorgesehen sind, deren Lichtaustrittsflächen beispielsweise an gegenüberliegenden Seiten einer Lichteintrittsfläche eines Beobachtungsstrahlengang angeordnet sind. Jeder einzelne Lichtleiter 30 weist einen geringeren Querschnitt auf als der Lichtleiter bei der Ausführungsform der Figuren 10 und 11. Jeder einzelne Lichtleiter 30 weist deshalb eine höhere Elastizität auf bzw. ist durch eine geringere Kraft verformbar. Ferner treten geringere mechanische Spannungen und weniger Reibung innerhalb der einzelnen Lichtleiter 30 auf.

Im Vergleich der Figuren 12 und 13 ist erkennbar, dass die erforderlichen Krümmungsradien der Lichtleiter 30 groß bzw. die erforderlichen Krümmungen gering sind. Insbesondere kann der erforderliche Krümmungsradius selbst bei einem großen Schwenkbereich größer sein als der Radius des Schafts. Dies reduziert in den beim Verändern der Beleuchtungsrichtung verformten Bereichen der Lichtleiters 30 das Risiko eines Bruchs oder anderer Schäden.

Bei der Darstellung der Ausführungsformen der Figuren 2 bis 8 wurde auf Einrichtungen zum Verstellen bzw. Einstellen der Blickrichtung, insbesondere der Führungseinrichtungen 50, 60 nicht näher eingegangen. Eine Kraftübertragungseinrichtung ähnlich den Kraftübertragungseinrichtungen, die oben anhand der Figuren 10 bis 13 dargestellt wurden, kann insbesondere auch bei der Ausführungsform der Figuren 2 bis 5 vorgesehen werden. Dabei ist die Kraftübertragungseinrichtung insbesondere nahe dem distalen Ende des Schlittens 58 bzw. nahe der Fassung 53 des Lichtleiters 30 mit dem Schlitten 58 verbunden, um bei der Verstellung der Blickrichtung 34, 35 einen großen Winkelbereich zuzulassen.

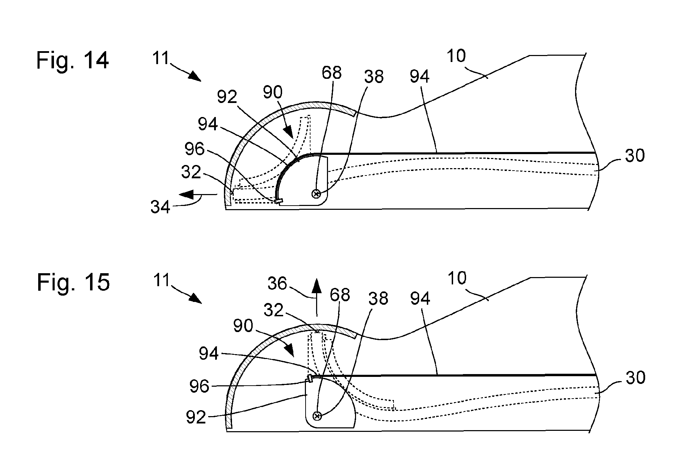

Die Figuren 14 und 15 zeigen schematische Darstellungen einer Einrichtung 90 zum Übertragen einer Kraft zu einer bewegbaren Lichtaustrittsfläche 32 oder einer anderen Lichtaustrittseinrichtung am distalen Ende 11 eines Endoskops 10 und zum Bewegen der Lichtaustrittseinrichtung zum Einstellen der Beleuchtungsrichtung 34, 36. Die Zeichenebenen der Figuren 14 und 15 sind parallel zu den Zeichenebenen der Figuren 2 bis 4, 6, 7 und 10 bis 13. Das distale Ende 11 des Endoskops 10 ist in den Figuren 14 und 15 bei zwei verschiedenen Beleuchtungsrichtungen 34, 36 dargestellt. Beispielhaft sind in gestrichelten Linien Einrichtungen und Merkmale des Ausführungsbeispiels der Figuren 6 bis 8 gezeigt. Für eine Beschreibung der in den Figuren 14 und 15 gestrichelt dargestellten Einrichtungen und Merkmale wird auf die Darstellung der Ausführungsform der Figuren 6 bis 8 verwiesen.

Die Einrichtung 90 umfasst eine Scheibeneinrichtung 92, die an einer Welle 68 befestigt ist. Die Scheibeneinrichtung 92 ist mit der Welle 68 um eine Schwenkachse 38 schwenkbar. Mit der Welle 68 ist insbesondere die in den Figuren 14 und 15 nur gestrichelt angedeutete Lichtaustrittsfläche 32 mechanisch gekoppelt. Eine Schwenkbewegung der Scheibeneinrichtung 92 hat deshalb eine entsprechende Schwenkbewegung der Lichtaustrittsfläche und der Beleuchtungsrichtung 34, 36 zur Folge. Die Scheibeneinrichtung 92 weist einen kreisbogenförmigen Randabschnitt auf, dessen Krümmungsmittelpunkt auf der Schwenkachse 38 liegt.

Im Schaft des Endoskops 10 erstreckt sich eine Riemeneinrichtung 94 vom proximalen Ende 12 des Endoskops 10 bis zum distalen Ende 11 des Endoskops 10. Die Riemeneinrichtung 94 umfasst insbesondere einen Draht oder ein Band aus Metall oder Kunststoff und weist eine geringe Dehnungselastizität auf. Das distale Ende der Riemeneinrichtung 94 ist mittels einer Befestigungseinrichtung 96 mit der Scheibeneinrichtung 92 mechanisch verbunden.

In der Zusammenschau der Figuren 14 und 15 ist erkennbar, dass eine Bewegung der Riemeneinrichtung 94 in Längsrichtung des Schafts des Endoskops 10 eine Schwenkbewegung der Scheibeneinrichtung 92, der Lichtaustrittsfläche 32 und der Beleuchtungsrichtung 34, 36 bewirkt. Bei einer Schwenkbewegung der Scheibeneinrichtung 92 im Uhrzeigersinn wird ein distaler Bereich der Riemeneinrichtung 94 von dem kreisbogenförmigen Randabschnitt der Scheibeneinrichtung 92 abgehoben. Bei einer Schwenkbewegung der Scheibeneinrichtung 92 gegen den Uhrzeigersinn wird ein distaler Bereich der Riemeneinrichtung 94 auf den kreisbogenförmigen Randabschnitt aufgelegt bzw. aufgewickelt.

Die Riemeneinrichtung 94 ist insbesondere lediglich bei Zugspannung steif und kann keine Schubkräfte übertragen. Damit eine Schwenkbewegung der Scheibeneinrichtung 92, der Lichtaustrittsfläche 32 und der Beleuchtungsrichtung 34, 36 gegen den Uhrzeigersinn trotzdem möglich ist, ist insbesondere eine Feder oder ein anderes elastisches Element am distalen Ende 11 des Endoskops 10 vorgesehen, das in den Figuren 14 und 15 nicht dargestellt ist. Diese Feder oder dieses andere elastische Element spannt die Scheibeneinrichtung 92 und die Lichtaustrittsfläche 32 in Richtung zu der in Figur 14 dargestellten Position vor.

### Bezugszeichen

- 10: Endoskop
- 11: distales Ende des Endoskops 10
- 12: proximales Ende des Endoskops 10
- 14: Schaft des Endoskops 10
- 15: erste Kupplung
- 16: zweite Kupplung
- 18: Längsachse des Endoskops 10
- 20: Fensterbauteil
- 21: erste extreme Blickrichtung (0°)
- 22: zweite extreme Blickrichtung (120°)
- 24: Fensterbauteil
- 26: Trennwand
- 28: Schwenkachse der Blickrichtung
- 29: Winkelbereich der Blickrichtungen
- 30: Lichtleiter
- 31: elastischer Abschnitt des Lichtleiters 30
- 32: Lichtaustrittsfläche des Lichtleiters 30
- 34: erste Beleuchtungsrichtung
- 35: zweite Beleuchtungsrichtung
- 36: dritte Beleuchtungsrichtung
- 38: Schwenkachse der Beleuchtungsrichtung
- 39: stark gekrümmter Abschnitt des Lichtleiters 30
- 41: lichtleitende Faser
- 42: Beschichtung der Faser 42
- 44: flexibler Mantel
- 46: Gleitmittel
- 50: Führungseinrichtung
- 52: Schelle zur Befestigung des Lichtleiters 30 am Schlitten 58
- 53: Fassung der Führungseinrichtung 50 für Lichtleiter 30
- 54: Kraftübertragungseinrichtung zum Übertragen einer Schub- oder Zugkraft
- 55: Längssicke der Kraftübertragungseinrichtung 54
- 56: Anlageeinrichtung der Führungseinrichtung 50
- 58: Schlitten der Führungseinrichtung 50
- 59: Führungsnut für Schlitten 58
- 60: Führungseinrichtung
- 63: Fassung der Führungseinrichtung 60 für Lichtleiter 30
- 66: Anlageeinrichtung der Führungseinrichtung 60
- 68: Welle der Führungseinrichtung 60
- 69: Ende der Welle 68
- 80: Kamera
- 88: Welle der Kamera 80
- 90: Einrichtung zum Bewegen der Lichtaustrittsfläche 32
- 92: Scheibeneinrichtung
- 94: Riemeneinrichtung
- 96: Befestigungseinrichtung zur Befestigung des distalen Endes der Riemeneinrichtung 94 an der Scheibeneinrichtung 92

## Patentansprüche

1. **Endoskop** (10) **mit einstellbarer Blickrichtung** (21, 22), mit:
einer **Lichtaustrittseinrichtung** (32) am distalen Ende (11) des Endoskops (10) zum Abstrahlen von Beleuchtungslicht in eine Beleuchtungsrichtung, wobei die Lichtaustrittseinrichtung (32) zum Einstellen der Beleuchtungsrichtung (34, 35, 36) relativ zum Endoskop (10) **bewegbar** ist;
einem **Lichtleiter** (30) zum Übertragen von Beleuchtungslicht zur Lichtaustrittseinrichtung (32),
wobei ein flexibler Abschnitt (31) des Lichtleiters (30) dazu ausgebildet und angeordnet ist, **elastisch verformt** zu werden, wenn die Lichtaustrittseinrichtung (32) bewegt wird,
**dadurch gekennzeichnet dass** das Endoskop ferner beinhaltet
eine **Kulissenführung** (58, 59, 50) zum Führen der Lichtaustrittseinrichtung (32),
die einen Schlitten (58) mit einer Fassung (53) und eine kreisbogenförmige Anlageeinrichtung (56) für den Lichtleiter (30) umfasst,
wobei die Anlageeinrichtung (56) starr mit dem Schlitten (58) verbunden ist,
wobei der Schlitten (58) in einer oder mehreren Führungsnuten oder an einem oder mehreren Stegen oder Schienen des distalen Endes des Endoskops geführt ist,
wobei die Führungsnuten, Stege oder Schienen bogenförmig oder kreisbogenförmig ausgeführt sind,
wobei ein distales Ende des Lichtleiters (30) nahe einer Lichtaustrittsfläche (32) des Lichtleiters (30) durch die Fassung (53) starr an dem Schlitten (58) gehalten ist,
einer **Kraftübertragungseinrichtung** (54) zum Übertragen einer Schub- oder Zugkraft zum proximalen Ende des Endoskops zum Bewegen der Lichtaustrittseinrichtung (32),
wobei die Kraftübertragungseinrichtung (54) mit dem Schlitten (58) der Kulissenführung **einstückig** ausgebildet ist.

2. Endoskop (10) nach dem vorangehenden Anspruch, bei dem der Lichtleiter (30) ein Bündel von lichtleitenden Fasern (41) umfasst, ferner mit:
einem **flexiblen Mantel** (44), in dem das Bündel von lichtleitenden Fasern (41) im Bereich des flexiblen Abschnitts (31) angeordnet ist.

3. Endoskop (10) nach dem vorangehenden Anspruch, ferner mit einem **Gleitmittel** (46), das in dem Mantel (44) angeordnet ist, um zumindest entweder Reibung innerhalb des Bündels von lichtleitenden Fasern (41) oder Reibung zwischen lichtleitenden Fasern (41) und dem flexiblen Mantel (44) zu mindern.

4. Endoskop (10) nach einem der vorangehenden Ansprüche, bei dem der Lichtleiter (30) ein Bündel aus lichtleitenden Fasern (41) umfasst, wobei lichtleitende Fasern (41) des Bündels eine **Beschichtung** (42) zur Herabsetzung von **Reibung** zwischen den lichtleitenden Fasern (41) aufweisen.

5. Endoskop (10) nach einem der vorangehenden Ansprüche, bei dem der Lichtleiter (30) ein Bündel von lichtleitenden Fasern (41) umfasst, wobei das Bündel einen **nicht-kreisförmigen** Querschnitt aufweist.

6. Endoskop (10) nach einem der vorangehenden Ansprüche, wobei das Endoskop (10) eine **Mehrzahl von Lichtleitern** (30) umfasst, die jeweils einen flexiblen Abschnitt (31) aufweisen, wobei der flexible Abschnitt (31) jedes Lichtleiters (30) dazu ausgebildet und angeordnet ist, elastisch verformt zu werden, wenn die Lichtaustrittseinrichtung (32) bewegt wird.

7. Endoskop (10) nach einem der vorangehenden Ansprüche, bei dem der Lichtleiter (30) an der Kraftübertragungseinrichtung mechanisch geführt ist.

## Claims

1. Endoscope (10) with an adjustable viewing direction (21, 22), comprising:
a light-emergence apparatus (32) at the distal end (11) of the endoscope (10) for radiating illumination light in an illumination direction, wherein the light-emergence apparatus (32) is movable relative to the endoscope (10) for setting the illumination direction (34, 35, 36);
an optical waveguide (30) for transmitting illumination light to the light-emergence apparatus (32),
wherein a flexible portion (31) of the optical waveguide (30) is embodied and arranged to be elastically deformed when the light-emergence apparatus (32) is moved,
**characterized in that** the endoscope furthermore contains
a sliding-block guide (58, 59, 50) for guiding the light-emergence apparatus (32),
which comprises a sled (58) with a holder (53) and a circular arc-shaped abutment apparatus (56) for the optical waveguide (30), wherein the abutment apparatus (56) is rigidly connected to the sled (58), wherein the sled is guided in one or more guide grooves or on one or more webs or rails of the distal end of the endoscope,
wherein the guide grooves, webs or rails are embodied in an arc-shaped or circular arc-shaped manner,
wherein a distal end of the optical waveguide (30) is held rigidly against the sled (58) in the vicinity of the light emergence face (32) of the optical waveguide (30) by way of the holder (53),
a force transmission apparatus (54) for transmitting a shearing or tensile force to the proximal end of the endoscope for moving the light-emergence apparatus (32),
wherein the force transmission apparatus (54) is formed integrally with the sled (58) of the sliding-block guide.

2. Endoscope (10) according to the preceding claim, in which the optical waveguide (30) comprises a bundle of light-guiding fibres (41), furthermore comprising:
a flexible jacket (44), in which the bundle of light-guiding fibres (41) is arranged in the region of the flexible portion (31).

3. Endoscope (10) according to the preceding claim, further comprising a lubricant (46), which is arranged in the jacket (44) in order, at least, to reduce either the friction within the bundle of light-guiding fibres (41) or the friction between light-guiding fibres (41) and the flexible jacket (44).

4. Endoscope (10) according to one of the preceding claims, wherein the optical waveguide (30) comprises a bundle of light-guiding fibres (41), wherein light-guiding fibres (41) of the bundle have a coating (42) for reducing friction between the light-guiding fibres (41).

5. Endoscope (10) according to one of the preceding claims, wherein the optical waveguide (30) comprises a bundle of light-guiding fibres (41), wherein the bundle has a non-circular cross section.

6. Endoscope (10) according to one of the preceding claims, wherein the endoscope (10) comprises a plurality of optical waveguides (30), which each have a flexible portion (31), wherein the flexible portion (31) of each optical waveguide (30) is embodied and arranged to be elastically deformed when the light-emergence apparatus (32) is moved.

7. Endoscope (10) according to one of the preceding claims, wherein the optical waveguide (30) is mechanically guided at the force transmission apparatus.

## Revendications

1. Endoscope (10) à direction d'observation réglable (21, 22), comportant :
un dispositif de sortie de lumière (32) à l'extrémité distale (11) de l'endoscope (10) destiné à émettre de la lumière d'éclairement dans une direction d'éclairement, dans lequel le dispositif de sortie de lumière (32) est mobile par rapport à l'endoscope (10) pour régler la direction d'éclairement (34, 35, 36) ;
un guide de lumière (30) destiné à transmettre de la lumière d'éclairement vers le dispositif de sortie de lumière (32),
dans lequel une partie flexible (31) du guide de lumière (30) est configurée et disposée de manière à ce qu'elle soit déformée élastiquement lorsque le dispositif de sortie de lumière (32) est déplacé,
**caractérisé en ce que** l'endoscope comporte en outre un guide à coulisse (58, 59, 50) destiné à guider le dispositif de sortie de lumière (32),
qui comprend un chariot (58) muni d'une monture (53) et d'un dispositif de butée (56) en forme d'arc de cercle destiné au guide de lumière (30),
dans lequel le dispositif de butée (56) est relié de manière rigide au chariot (58),
dans lequel le chariot (58) est guidé dans une ou plusieurs gorges de guidage ou sur une ou plusieurs nervures ou un ou plusieurs rails de l'extrémité distale de l'endoscope,
dans lequel les gorges de guidage, les nervures ou les rails sont réalisés de manière à présenter la forme d'arcs ou d'arcs de cercles,
dans lequel une extrémité distale du guide de lumière (30) est maintenue de manière rigide par l'intermédiaire de la monture (53) sur le chariot (58) à proximité d'une surface de sortie de lumière (32) du guide de lumière (30),
un dispositif de transmission de force (54) destiné à transmettre une force de poussée ou de traction à l'extrémité proximale de l'endoscope pour déplacer le dispositif de sortie de lumière (32),
dans lequel le dispositif de transmission de force (54) est réalisé de manière solidaire du chariot (58) du guide à coulisse.

2. Endoscope (10) selon la revendication précédente, dans lequel le guide de lumière (30) comprend un faisceau de fibres guidant la lumière (41), comportant en outre :
une gaine flexible (44), dans laquelle le faisceau de fibres guidant la lumière (41) est disposé dans la région de la partie flexible (31).

3. Endoscope (10) selon l'une quelconque des revendications précédentes, comportant en outre un lubrifiant (46) qui est disposé dans la gaine (44) pour au moins réduire soit le frottement se produisant à l'intérieur du faisceau de fibres guidant la lumière (41), soit le frottement se produisant entre des fibres guidant la lumière (41) et la gaine flexible (44).

4. Endoscope (10) selon l'une quelconque des revendications précédentes, dans lequel le guide de lumière (30) comprend un faisceau de fibres guidant la lumière (41), dans lequel les fibres guidant la lumière (41) du faisceau présentent un revêtement (42) destiné à réduire le frottement entre les fibres guidant la lumière (41).

5. Endoscope (10) selon l'une quelconque des revendications précédentes, dans lequel le guide de lumière (30) comprend un faisceau de fibres guidant la lumière (41), dans lequel le faisceau présente une section transversale de forme non circulaire.

6. Endoscope (10) selon l'une quelconque des revendications précédentes, dans lequel l'endoscope (10) comprend une pluralité de guides de lumière (30) qui présentent respectivement une partie flexible (31), dans lequel la partie flexible (31) de chaque guide de lumière (30) est réalisée et disposée de manière à ce qu'elle soit déformée élastiquement lorsque le dispositif de sortie de lumière (32) est déplacé.

7. Endoscope (10) selon l'une quelconque des revendications précédentes, dans lequel le guide de lumière (30) est guidé mécaniquement sur le dispositif de transmission de force.
